# EUROPÄISCHE PATENTSCHRIFT

(11) **EP 0 618 912 B1**
(45) Veröffentlichungstag und Bekanntmachung des Hinweises auf die Patenterteilung: **20.11.1996**
(21) Anmeldenummer: 93901680.4
(22) Anmeldetag: 15.12.1992
(51) Int. Cl.: C07D 285/34, A01N 43/88

(54) **VERFAHREN ZUR HERSTELLUNG EINES WEITGEHEND STAUBFREIEN TETRAHYDRO-3,5-DIMETHYL-1,3,5-THIADIAZIN-2-THION GRANULATS**
METHOD OF PRODUCING TETRAHYDRO-3,5-DIMETHYL-1,3,5-THIADIAZIN-2-THION POWDER SUBSTANTIALLY FREE OF DUST FINES
PROCEDE DE FABRICATION DE GRANULATS DE TETRAHYDRO-3,5-DIMETHYL-1,3,5-THIADIAZINE-2-THIONE PRATIQUEMENT SANS POUSSIERES

(30) Priorität: 21.12.1991 DE 4142571
(43) Veröffentlichungstag der Anmeldung: 12.10.1994
(73) Patentinhaber: BASF Aktiengesellschaft, 67063 Ludwigshafen (DE)
(72) Erfinder: APPLER, Heinz, D-8952 Marktoberdorf-Balteratsried (DE)
(86) Internationale Anmeldenummer: EP9202907
(87) Internationale Veröffentlichungsnummer: WO9313085

(56) Entgegenhaltungen:
- FR-A- 1 229 662
- FR-A- 1 554 038
- US-A- 2 838 389
- CHEMICAL ABSTRACTS, vol. 102, no. 19, 13. Mai 1985, Columbus, Ohio, US; abstract no. 166783g, Seite 624 ; in der Anmeldung erw hnt

## Beschreibung

Die vorliegende Erfindung betrifft ein Verfahren zur Herstellung eines weitgehend staubfreien Granulats von Tetrahydro-3,5-dimethyl-1,3,5-thiadiazin-2-thion (I) durch Umsetzung von Methylamin (II) mit Schwefelkohlenstoff (III) und Formaldehyd (IV) oder durch Umsetzung des Methylammonium-Salzes der N-Methyl-dithiocarbaminsäure (V) mit Formaldehyd (IV).

Außerdem betrifft die Erfindung ein weitgehend staubfreies Granulat von Tetrahydro-3,5-dimethyl-1,3,5-thiadiazin-2-thion sowie Verfahren zur Bodenentseuchung bzw. zur Bekämpfung von Nematoden keimenden Pflanzen und Bodenpilzen mit diesem Granulat.

Tetrahydro-3,5-dimethyl-1,3,5-thiadiazin-2-thion (I) (Kurzbezeichnung:Dazomet) wird in der Landwirtschaft und Gärtnerei zur Bodenentseuchung (gegen Nematoden, keimende Pflanzen und Bodenpilze) eingesetzt (US-A 2,838,389).

Nach den bekannten Herstellungsverfahren, wie sie z. B. aus FR-A-1 554 038 und FR-A-1 229 662 bekannt sind, fällt der Wirkstoff in Form eines feinen Pulvers an, welches zudem einen hohen Anteil an Wirkstoff als Staub enthält. Im Hinblick auf eine sichere Anwendung des Wirkstoffs, der bei der Zersetzung Methyl-Isothiocyanat freisetzt, ist ein derartiges Produkt nicht geeignet.

Aus der Literatur ist bekannt, daß man Thiadiazinderivate wie Tetrahydro-3,5-dimethyl-1,3,5-thiadiazin-2-thion (I) als Granulat erhält, wenn man die Umsetzung der Edukte in Gegenwart eines Emulgators (Emulgen PP150) und Zinksulfat durchführt (JP-A 84/210 073 = Chemical Abstracts 102(19), 166 783 g). Das so hergestellte Granulat enthält 10 % an Körnern eines Durchmessers von 200 bis 300 µ, 79 % an Körnern eines Durchmessers von 100 bis 200 µ und 11 % an Körnern eines Durchmessers von weniger als 100 µ.

Durch die Verwendung des anorganischen Salzes und des Emulgators können bei diesem Verfahren jedoch Probleme bei der Entsorgung der wäßrigen Mutterlaugen auftreten.

Demgemäß war die Aufgabe der vorliegenden Erfindung ein einfacheres Verfahren zur Herstellung von Tetrahydro-3,5-dimethyl-1,3,5-thiadiazin-2-thion (I) als Granulat.

Entsprechend dieser Aufgabe wurde ein Verfahren zur Herstellung eines weitgehend staubfreien Granulats von Tetrahydro-3,5-dimethyl-1,3,5-thiadiazin-2-thion (I) durch Umsetzung von Methylamin (II) mit Schwefelkohlenstoff (III) und Formaldehyd (IV) oder durch Umsetzung des Methylammonium-Salzes der N-Methyl-dithiocarbaminsäure (V) mit Formaldehyd (IV) gefunden, welches dadurch gekennzeichnet ist, daß man die Umsetzung in Gegenwart von 0,1 mol-% bis 10 mol-%, bezogen auf II, mindestens eines Diaminoalkylens der Formel VI

R¹-NH-A-NH-R² VI

in der R¹ und R² unabhängig voneinander für Wasserstoff oder eine C₁-C₄-Alkylgruppe stehen und A eine 1,2-Ethylen-, eine 1,3-Propylen- oder eine 1,4-Butylenbrücke bedeutet, wobei diese Brücken ein bis vier C₁-C₄-Alkylgruppen tragen können, durchführt .

Die Umsetzung erfolgt nach dem folgenden Reaktionsschema: 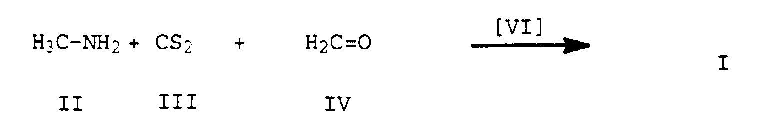 oder

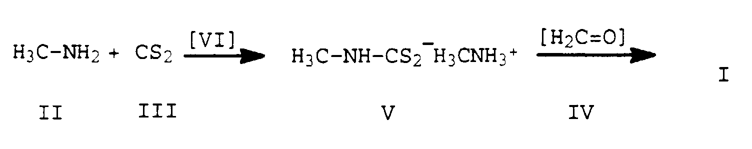

Das Verfahren könnte darauf beruhen, daß geringe Mengen der Diaminoalkylenverbindung bei der Umsetzung mit Methylamin in Konkurrenz treten und so - sofern die Reste R¹ und R² gleichzeitig Wasserstoff bedeuten - beispielsweise "Dimere" der Formel VII oder höhere "Polymere" des Wirkstoffs gebildet werden können.

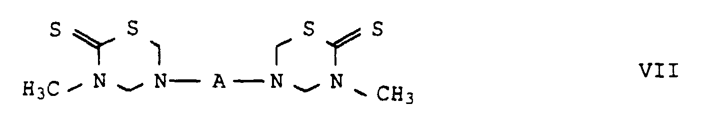

Mit den Diaminoalkylenverbindungen, in denen R¹ oder R² nicht Wasserstoff bedeuten, könnten sich beispielsweise Nebenprodukte der folgenden Struktur VIII bilden.

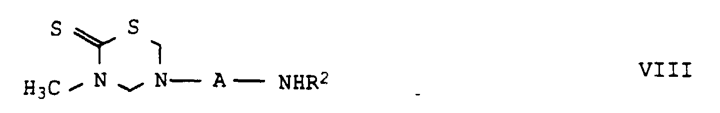

Diaminoalkylenverbindungen, in denen weder R¹ noch R² Wasserstoff bedeutet, könnten möglicherweise mit Schwefelkohlenstoff zu nicht-cyclisierten Produkten der Struktur IX reagieren.

R¹-N⁺H₂-A-NR²-CS₂- IX

Neben den vorstehend skizzierten möglichen Nebenprodukten sind auch andere Strukturen denkbar.

Von wesentlicher Bedeutung ist jedoch, daß auch die denkbaren Nebenprodukte wie der Wirkstoff selbst in der Lage wären, Methyl-Isothiocyanat freizusetzen. Auch die Nebenprodukte könnten somit im Granulat zur Wirkung beitragen.

Die Bildung des gewünschten Granulats könnte darauf zurückzuführen sein, daß Nebenprodukte der vorstehend angenommenen Strukturen einerseits zwar ausreichen, um die "geordnete" Kristallisation zu stören, auf der anderen Seite jedoch derartige Verbindungen genügend Ähnlichkeit mit dem Wirkstoff selbst aufweisen, so daß sie ein ungeordnetes Konglomerat mit den Kristallen und somit das gewünschte Granulat bilden.

Das erfindungsgemäße Verfahren wird üblicherweise in wäßriger Lösung entweder in einer einstufigen Synthese oder in zwei Stufen durchgeführt.

Bei der einstufigen Reaktionsführung geht man im allgemeinen so vor, daß man eine wäßrige Lösung von Methylamin und Diaminoalkylen zunächst mit Schwefelkohlenstoff versetzt und daran anschließend eine wäßrige Formaldehyd-Lösung zugibt (in Analogie zu dem in der US-A 2,838,389 (Spalte 6, Zeilen 46-57) beschriebenen Verfahren).

Bei der Umsetzung in zwei Reaktionsstufen geht man im allgemeinen so vor, daß man zunächst eine wäßrige Lösung von Methylamin und Diaminoalkylen mit Schwefelkohlenstoff versetzt, anschließend die Lösung des gebildeten Carbamats V von überschüssigem Schwefelkohlenstoff befreit und diese so vorgereinigte Lösung zu einer wäßrigen Formaldehyd-Lösung gibt.

Da die Umsetzungen exotherm verlaufen, andererseits aber sowohl das Zwischenprodukt als auch der Wirkstoff thermisch instabil sind, empfiehlt es sich die Reaktionstemperatur durch Kühlung herabzusetzen.

Im allgemeinen verlaufen die Umsetzungen oberhalb von 10°C mit ausreichender Geschwindigkeit. Bei Temperaturen von mehr als 50°C kommt es bereits merklich zur Bildung von unerwünschten Zersetzungsprodukten. Aus diesem Grund werden die Umsetzungen üblicherweise bei Temperaturen von 10 bis 40°C, vorzugsweise 15 bis 30°C, durchgeführt.

Während man bei der einstufigen Reaktionsführung die Edukte II und III möglichst in stöchiometrischen Mengen miteinander umsetzt, verwendet man bei der Synthese über zwei Stufen im allgemeinen einen Überschuß an Schwefelkohlenstoff (III).

Unabhängig von der Reaktionsführung verwendet man den Formaldehyd üblicherweise ebenfalls in einem geringen überschuß bezogen auf die Menge an Methylamin (II).

Im Hinblick auf die erfindungsgemäße Verwendung kommen Diaminoalkylenverbindungen der Formel VI in Betracht, in denen die Substituenten die folgende Bedeutung haben:
R¹ und R² unabhängig voneinander

Wasserstoff und C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Wasserstoff, Methyl und Ethyl, insbesondere Wasserstoff und Methyl;
A
eine 1,2-Ethylen-, eine 1,3-Propylen- oder eine 1,4-Butylenbrücke, wobei diese Brücken eine bis vier C₁-C₄-Alkylgruppen wie vorstehend genannt, vorzugsweise eine oder zwei Methylgruppen, tragen können.

Bevorzugte Diaminoalkylenverbindungen der Formel VI sind: 1,2-Diaminoethylen, 1-(N-Methylamino)-2-aminoethylen, 1,2-Di-(N-methylamino)ethylen, 1,2-Diaminopropylen, 1-(N-Methylamino)-2-aminopropylen, 1,2-Di-(N-methylamino)propylen, 1,3-Diaminopropylen, 1-(N-Methylamino)-3-aminopropylen, 1,3-Di-(N-methylamino)propylen, 1,2-Diaminobutylen, 1-(N-Methylamino)-2-aminobutylen, 1,2-Di-(N-methylamino)butylen, 2,3-Diaminobutylen, 2-(N-Methylamino)-3-aminobutylen, 2,3-Di-(N-methylamino)butylen, 1,4-Diaminobutylen, 1-(N-Methylamino)-4-aminobutylen und 1,4-(N-methylamino)butylen.

Insbesondere bevorzugt ist die Verwendung von 1,2-Diaminoethylen, 1-(N-Methylamino)-2-aminoethylen, 1,2-Di-(N-methylamino)ethylen, 1,2-Diaminopropylen, 1,2-Di-(N-methylamino)propylen und 1-(N-Methylamino)-2-aminopropylen, wobei sowohl die reinen Verbindungen als auch Gemische dieser Verbindungen verwendet werden können.

Man setzt der Reaktionsmischung 0,1 mol-% bis 10 mol-% an Diaminoalkylen VI bezogen auf die Menge an eingesetztem Methylamin (II) zu, vorzugsweise verwendet man 0,2 mol-% bis 5 mol-%, insbesondere 0,5 mol-% bis 1,5 mol-%.

Durch die zusätzliche Zugabe von Impfkristallen kann bei diesem Prozeß außerdem in an sich bekannter Weise Einfluß auf die Größe der Granula genommen werden. So wären beispielsweise bei einem sehr hohen Anteil an Impfkristallen bezogen auf die Edukte kleine Granula zu erwarten, während ein sehr geringer Anteil an Impfkristallen größere Granula zur Folge hätte.

Als Impfkristalle verwendet man feinverteiltes Tetrahydro-3,5-dimethyl-1,3,5-thiadiazin-2-thion (I) in einer Menge von 1,5 mol-% bis 10 mol-%, vorzugsweise 2,5 mol-% bis 7,5 mol-%, insbesondere 3 mol-% bis 6 mol-%, bezogen auf V, wobei Impfkristalle mit einer Korngröße (Durchmesser) von weniger als 100 µ Verwendung finden. Üblicherweise sollten die Korngrößen zu etwa 90 % zwischen 50 und 5 µ liegen. Insbesondere bevorzugt ist eine Korngrößenverteilung, in der die Teilchen zu 100 % kleiner als 100 µ sind, zu etwa 90 % zwischen 50 und 5 µ liegen und zu etwa 10 % geringer als 5 µ sind.

Um vom Beginn der Zugabe der Impfkristalle an eine möglichst gleichmäßige Verteilung im Reaktionsmedium zu erreichen, setzt man die Impfkristalle dem Reaktionsmedium bevorzugt in Form einer wäßrigen Suspension zu.

Die Suspension der Impfkristalle wird sowohl bei der einstufigen Reaktionsführung als auch bei der Umsetzung in zwei Reaktionsstufen der wäßrigen Formaldehyd-Lösung beigemischt.

Die Größe der nach dem erfindungsgemäßen Verfahren erhältlichen Granula kann außer durch den Zusatz der Impfkristalle und die Menge an Diaminoalkylen VI noch
- durch die Geschwindigkeit der Zugabe der Reaktionspartner (Formaldehyd-Lösung bei einstufiger Reaktionsführung bzw. Carbamat-Lösung bei der Umsetzung in zwei Reaktionsstufen),
- durch die Stärke der Durchmischung der Reaktionspartner bei der Umsetzung und
- durch die Dauer der Durchmischung der Reaktionspartner nach beendeter Zugabe der Formaldehyd-Lösung bei einstufiger Reaktionsführung bzw. Carbamat-Lösung bei der Umsetzung in zwei Reaktionsstufen
beeinflußt werden, wobei diese Größen wegen ihrer Abhängigkeit von der Menge an Reaktionspartnern, wegen der Abhängigkeit von der Geometrie des Reaktionsgefäßes und wegen der Abhängigkeit von der Art der Durchmischung nach dem allgemeinen Fachwissen ermittelt werden müssen. Hierbei sind die folgenden allgemein bekannten Zusammenhänge zu beachten:
- Je höher die Geschwindigkeit der Zugabe der Reaktionspartner ist, desto kleiner sind die gebildeten Granula.
- Je stärker die Durchmischung der Reaktionspartner ist, desto kleiner sind die gebildeten Granula, wobei zusätzlich Abrieb-Effekte dazu führen können, daß das Produkt einen hohen Anteil an sehr feinem Produkt (Feinanteil) enthält, was nach dem Trocknen zu Produkt-Staub führen kann.
- Je länger die Durchmischung nach der Beendigung der Zugabe fortgesetzt wird, um so höher werden Abrieb-Effekte und damit der Feinanteil im Produkt.

Das nach dem erfindungsgemäßen Verfahren erhältliche Granulat eignet sich in der für den Wirkstoff an sich bekannten Weise zur Bodenentseuchung.

### Verfahrensbeispiele:

### Beispiel 1

Eine Mischung aus 111 g Methylamin, 4,37 g Ethylendiamin, 1,24 g N-Methylethylendiamin und 520 ml Wasser wurde unter Rühren bei 20 bis 30°C mit 140,5 g Schwefelkohlenstoff versetzt. Nach beendeter Zugabe wurde die Reaktionsmischung 2 h bei 25°C gerührt und anschließend mit Wasser auf ein Volumen von 800 ml aufgefüllt.

Die so erhaltene Lösung wurde dann bei 30 bis 50°C zu einer vorbereiteten Mischung aus 410 g einer 30 %-igen wäßrigen Formaldehyd-Lösung und 400 ml Wasser gegeben.

Das entstandene Tetrahydro-3,5-dimethyl-1,3,5-thiadiazin-2-thion-Granulat wurde von der Mutterlauge abgetrennt, gewaschen und getrocknet. Der Durchmesser der erhaltenen Granula lag zu 80 % im Bereich von 400 bis 500 µ.

### Beispiel 2

Analog zu der in Beispiel 1 angegebenen Verfahrensweise wurde zunächst bei 30 bis 50°C aus 293,7 g einer 40 %-igen Methylamin-Lösung (in Wasser, entsprechend 117,5 g Methylamin) und 2,25 g Ethylendiamin in 300 ml Wasser durch Zugabe von 157,8 g Schwefelkohlenstoff eine Dithiocarbamat-Lösung bereitet. Nach der Abtrennung von nicht umgesetztem Schwefelkohlenstoff wurde mit Wasser auf ein Volumen von 800 ml aufgefüllt.

Durch Zugabe der so erhaltenen Lösung zu einer Mischung von 322 g einer 40 %-igen wäßrigen Formaldehyd-Lösung, welche 15 g Impfkristalle (Korngrößenverteilung: 100 Gew.-% kleiner als 100 µ, ca. 90 Gew.-% zwischen 50 und 5 µ und ca. 10 Gew.-% kleiner als 5 µ) und 900 ml Wasser enthielt, erhielt man (nach Abtrennung, Reinigung und Trocknung) ein Tetrahydro-3,5-dimethyl-1,3,5-thiadiazin-2-thion Granulat bei dem der Durchmesser der Granula zu 100 Gew.-% kleiner als 400 µ war, ca. 90 Gew.-% der Teilchen hatten einen Durchmesser im Bereich von 400 bis 100 µ und bei weniger als 10 Gew.-% war der Durchmesser kleiner als 100 µ.

## Patentansprüche

1. Verfahren zur Herstellung eines weitgehend staubfreien Granulats von Tetrahydro-3,5-dimethyl-1,3,5-thiadiazin-2-thion (I) durch Umsetzung von Methylamin (II) mit Schwefelkohlenstoff (III) und Formaldehyd (IV) oder durch Umsetzung des Methylammonium-Salzes der N-Methyldithiocarbaminsäure (V) mit Formaldehyd (IV), dadurch gekennzeichnet, daß man die Umsetzung in Gegenwart von 0,1 mol-% bis 10 mol-%, bezogen auf (II), mindestens eines Diaminoalkylens der Formel VI
R¹-NH-A-NH-R² VI
in der R¹ und R² unabhängig voneinander für Wasserstoff oder eine C₁-C₄-Alkylgruppe stehen und A eine 1,2-Ethylen-, eine 1,3-Propylen- oder eine 1,4-Butylenbrücke bedeutet, wobei diese Brücken ein bis vier C₁-C₄-Alkylgruppen tragen können, dürchführt.

2. Verfahren nach Anspruch 1, dadurch gekennzeichnet, daß man die Umsetzung in Gegenwart von feinverteiltem Tetrahydro-3,5-dimethyl-1,3,5-thiadiazin-2-thion (I) durchführt.

3. Verfahren nach Anspruch 1, dadurch gekennzeichnet, daß man mindestens ein Diaminoalkylen der Formel VI, gemäß Anspruch 1, verwendet, wobei R¹ oder R² unabhängig voneinander für Wasserstoff, Methyl oder Ethyl stehen und A eine Ethylenbrücke bedeutet welche ihrerseits ein oder zwei Methyl- oder Ethylgruppen tragen kann.

4. Verfahren nach Anspruch 1, dadurch gekennzeichnet, daß man die Umsetzung in Gegenwart von 0,2 mol-% bis 5 mol-% an Diaminoalkylen der Formel VI gemäß Anspruch 1, bezogen auf II, durchführt.

5. Verfahren nach Anspruch 2, dadurch gekennzeichnet, daß man feinverteiltes Tetrahydro-3,5-dimethyl-1,3,5-thiadiazin-2-thion (I) in einer Menge von 1,5 mol-% bis 10 mol-% bezogen auf V verwendet.

6. Verfahren nach Anspruch 2, dadurch gekennzeichnet, daß das feinverteilte Tetrahydro-3,5-dimethyl-1,3,5-thiadiazin-2-thion (I) eine Korngröße von weniger als 100 µ hat.

7. Verfahren nach Anspruch 2, dadurch gekennzeichnet, daß man das feinverteilte Tetrahydro-3,5-dimethyl-1,3,5-thiadiazin-2-thion (I) in Form einer wäßrigen Suspension verwendet.

8. Weitgehend staubfreies Granulat von Tetrahydro-3,5-dimethyl-1,3,5-thiadiazin-2-thion erhältlich nach dem Verfahren gemäß Anspruch 1, dadurch gekennzeichnet, daß es Dimere oder höhere Polymere des Wirkstoffs enthält, wobei diese Dimeren oder höheren Polymere dadurch gekennzeichnet sind, daß zwei nicht zum gleichen Molekül I gehörende Stickstoffatome durch eine dem Substituenten A gemäß Anspruch 1 entsprechende 1,2-Ethylen-, 1,3-Propylen- oder 1,4-Butylen-Brücke miteinander verknüpft sind.

9. Verfahren zur Bodenentseuchung, dadurch gekennzeichnet, daß man den Boden mit einer wirksamen Menge des weitgehend staubfreien Granulats von Tetrahydro-3,5-dimethyl-1,3,5-thiadiazin-2-thion gemäß Anspruch 8 behandelt.

10. Verfahren zur Bekämpfung von Nematoden, keimenden Pflanzen und Bodenpilze, dadurch gekennzeichnet, daß man den Erdboden mit einer wirksamen Menge des weitgehend staubfreien Granulats von Tetrahydro-3,5-dimethyl-1,3,5-thiadiazin-2-thion gemäß Anspruch 8 behandelt.

11. Verwendung eines des weitgehend staubfreien Granulats von Tetrahydro-3,5-dimethyl-1,3,5-thiadiazin-2-thion gemäß Anspruch 8 zur Bodenentseuchung.

12. Verwendung eines des weitgehend staubfreien Granulats von Tetrahydro-3,5-dimethyl-1,3,5-thiadiazin-2-thion gemäß Anspruch 8 zur Bekämpfung von Nematoden, keimenden Pflanzen und Bodenpilzen.

## Claims

1. A process for the preparation of substantially dust-free granules of tetrahydro-3,5-dimethyl-1,3,5-thiadiazine-2-thione (I) by reacting methylamine (II) with carbon disulfide (III) and formaldehyde (IV) or by reacting the methylammonium salt of N-methyldithiocarbamic acid (V) with formaldehyde (IV), wherein the reaction is carried out in the presence of from 0.1 to 10 mol %, based on II, of at least one diaminoalkylene of the formula VI
R¹-NH-A-NH-R² VI
where R¹ and R² independently of one another are each hydrogen or C₁-C₄-alkyl and A is a 1,2-ethylene, 1,3-propylene or 1,4-butylene bridge, and these bridges may carry from one to four C₁-C₄-alkyl groups.

2. A process as claimed in claim 1, wherein the reaction is carried out in the presence of a finely divided tetrahydro-3,5-dimethyl-1,3,5-thiadiazine-2-thione (I).

3. A process as claimed in claim 1, wherein at least one diaminoalkylene of the formula VI as claimed in claim 1 is used, R¹ or R² independently of one another each being hydrogen, methyl or ethyl and A being an ethylene bridge which in turn may carry one or two methyl or ethyl groups.

4. A process as claimed in claim 1, wherein the reaction is carried out in the presence of from 0.2 to 5 mol %, based on II, of a diaminoalkylene of the formula VI as claimed in claim 1.

5. A process as claimed in claim 2, wherein the finely divided tetrahydro-3,5-dimethyl-1,3,5-thiadiazine-2-thione (I) is used in an amount from 1.5 to 10 mol %, based on V.

6. A process as claimed in claim 2, wherein the finely divided tetrahydro-3,5-dimethyl-1,3,5-thiadiazine-2-thione (I) has a particle size of less than 100 µm.

7. A process as claimed in claim 2, wherein the finely divided tetrahydro-3,5-dimethyl-1,3,5-thiadiazine-2-thione (I) is used in the form of an aqueous suspension.

8. Substantially dust-free granules of tetrahydro-3,5-dimethyl-1,3,5-thiadiazine-2-thione, obtainable by a process as claimed in claim 1, wherein the granules contain dimers or higher polymers of the active ingredient in which dimers or higher polymers two nitrogen atoms not belonging to the same molecule I are linked to one another by a 1,2-ethylene, 1,3-propylene or 1,4-butylene bridge corresponding to the substituent A as claimed in claim 1.

9. A method of soil decontamination, which comprises treating the soil with an effective amount of substantially dust-free granules of tetrahydro-3,5-dimethyl-1,3,5-thiadiazine-2-thione as claimed in claim 8.

10. A method of controlling nematodes, germinating plants and soil fungi, which comprises treating the earth with an effective amount of substantially dust-free granules of tetrahydro-3,5-dimethyl-1,3,5-thiadiazine-2-thione as claimed in claim 8.

11. The use of substantially dust-free granules of tetrahydro-3,5-dimethyl-1,3,5-thiadiazine-2-thione as claimed in claim 8 for soil decontamination.

12. The use of substantially dust-free granules of tetrahydro-3,5-dimethyl-1,3,5-thiadiazine-2-thione as claimed in claim 8 for controlling nematodes, germinating plants and soil fungi.

## Revendications

1. Procédé de préparation de granulés, pratiquement sans poussière, de tétrahydro-3,5-diméthyl-1,3,5-thiadiazine-2-thione I par réaction de la méthylamine II avec le sulfure de carbone III et le formaldéhyde IV ou par réaction du sel de méthylammonium de l'acide N-méthyldithiocarbamique V avec le formaldéhyde IV, caractérisé par le fait que l'on effectue la réaction en présence de 0,1 à 10 mol %, par rapport à II, d'au moins un diaminoalkylène de formule VI
R¹-NH-A-NH-R² VI
dans laquelle R¹ et R² représentent chacun, indépendamment l'un de l'autre, l'hydrogène ou un groupe alkyle en C1-C4 et A représente un pont 1,2-éthylène, 1,3-propylène ou 1,4-butylène, ces ponts pouvant porter un à quatre groupes alkyle en C1-C4.

2. Procédé selon la revendication 1, caractérisé par le fait que l'on effectue la réaction en présence d'une tétrahydro-3,5-diméthyl-1,3,5-thiadiazine-2-thione I à l'état de fine division.

3. Procédé selon la revendication 1, caractérisé par le fait que l'on utilise au moins un diaminoalkylène de formule VI de la revendication 1 dans laquelle R¹ ou R² représentent chacun, indépendamment l'un de l'autre, l'hydrogène, un groupe méthyle ou éthyle et A représente un pont d'éthylène qui peut lui-même porter un ou deux groupes méthyle ou éthyle.

4. Procédé selon la revendication 1, caractérisé par le fait que l'on effectue la réaction en présence de 0,2 à 5 mol % d'un diaminoalkylène de formule VI de la revendication 1, par rapport à II.

5. Procédé selon la revendication 2, caractérisé par le fait que l'on utilise la tétrahydro-3,5-diméthyl-1,3,5-thiadiazine-2-thione I à l'état de fine division en quantité de 1,5 mol % à 10 mol % par rapport à V.

6. Procédé selon la revendication 2, caractérisé par le fait que la tétrahydro-3,5-diméthyl-1,3,5-thiadiazine-2-thione I à l'état de fine division est à une dimension de grain inférieure à 100 µm.

7. Procédé selon la revendication 2, caractérisé par le fait que l'on utilise la tétrahydro-3,5-diméthyl-1,3,5-thiadiazine-2-thione I à l'état de fine division sous forme d'une suspension aqueuse.

8. Granulés pratiquement sans poussière de tétrahydro-3,5-diméthyl-1,3,5-thiadiazine-2-thione obtenus par le procédé selon la revendication 1, caractérisés par le fait qu'ils contiennent le dimère ou des polymères supérieurs de la substance active, ces dimères ou polymères supérieurs se caractérisant par le fait que deux atomes d'azote n'appartenant pas à la même molécule I sont reliés entre eux par un pont 1,2-éthylène, 1,3-propylène ou 1,4-butylène correspondant au substituant A de la revendication 1.

9. Procédé pour désinfecter le sol, caractérisé par le fait qu'on traite par une quantité efficace des granulés pratiquement sans poussière de tétrahydro-3,5-diméthyl-1,3,5-thiadiazine-2-thione selon la revendication 8.

10. Procédé pour combattre les nématodes, les plantes qui germent et les mycètes du sol, caractérisé par le fait que l'on traite la terre par une quantité efficace des granulés pratiquement sans poussière de tétrahydro-3,5-diméthyl-1,3,5-thiadiazine-2-thione selon la revendication 8.

11. Utilisation des granulés pratiquement sans poussière de tétrahydro-3,5-diméthyl-1,3,5-thiadiazine-2-thione selon la revendication 8 pour la désinfection du sol.

12. Utilisation des granulés pratiquement sans poussière de tétrahydro-3,5-diméthyl-1,3,5-thiadiazine-2-thione selon la revendication 8 pour la lutte contre les nématodes, les plantes qui germent et les mycètes du sol.
